# EUROPEAN PATENT SPECIFICATION

(11) **EP 3 233 020 B1**
(45) Date of publication and mention of the grant of the patent: **06.05.2020**
(21) Application number: 15816334.5
(22) Date of filing: 08.12.2015
(51) Int. Cl.: A61K 8/02, A61K 8/31, A61K 8/58, A61K 8/86, A61K 8/891, A61Q 9/02, B26B 21/44, C10M 169/04

(54) **LUBRICATING MEMBERS FOR RAZOR CARTRIDGES**
GLEITMITTELSTREIFEN FÜR RASIERKLINGENEINHEITEN
ÉLÉMENTS DE LUBRIFICATION POUR CARTOUCHES DE RASOIR

(30) Priority: 18.12.2014 US 201462093739 P
(43) Date of publication of application: 25.10.2017
(73) Proprietor: The Gillette Company LLC, Boston, MA 02127 (US)
(72) Inventor: MOLONEY, Michael, John, Boston, Massachusetts 02127 (US); HAINES, Christopher, Boston, Massachusetts 02127 (US); STEPHENS, Alison, Fiona, Egham Surrey TW20 9NW (GB); LU, Bailu, Reading Berkshire RG2 0QE (GB)
(74) Representative: Hoyng Rokh Monegier LLP
(86) International application number: PCT/US2015/064411
(87) International publication number: WO 2016/100001

(56) References cited:
- US-A- 4 170 821
- US-A- 5 431 906
- US-A1- 2014 323 374

## Description

### FIELD OF THE INVENTION

The invention relates to lubricating members for razor cartridges comprising a water soluble polymer exhibiting improved lubricating properties which can be readily manufactured without impacting performance

### BACKGROUND OF THE INVENTION

The use of shaving aids on razor blades to provide lubrication benefits during the shave is known. *See e.g.,* US7,121,754; US6,298,558; US5,711,076; US5,134,775; US 4170821; US6,301,785, U.S. 2009/0223057 and US 2006/0225285. Such shaving aids typically comprise a water-insoluble matrix material to provide structural integrity and a water-soluble polymer such as polyethylene oxide (polyox) in order to provide the lubrication during the shave once the water-soluble polymer forms a solution with the water present during shaving. Since the introduction of polyox as a shaving lubricant, however little development has been made in the field, even though polyethylene oxide polymers are not without limitations. For example, the use of polyethylene oxide polymers having a low molecular weight only provides limited lubrication, and while improved lubrication may be seen when using polyethylene oxide polymer having higher molecular weights, this negatively impacts other aspects of the aqueous solution typically formed in-use. For example, the resultant viscosity in aqueous solution may also increase, leading to negatively perceived attributes, for example concerning the feeling of the shave for the user, particularly in respect of the lubricant. The prior art does also describe the use of combinations of high and low molecular weight polyethylene oxide polymers in order to balance these performance attributes. Nevertheless, such combinations are also limited in their ability to improve performance and or suffer from other negative performance attributes. US2014 0323374 describes the combination of a water soluble polymer having a molecular weight of at least 5000 and a copolymer of polyethylene oxide and polypropylene oxide to address this problem. The art further describes the incorporation of additional materials to further improve the lubrication performance. For example US6442839, US 5431906, US2007/0110703 and US2009/0223057 describe the use of low levels of mineral and essential oils, butters, waxes and silicones. The use of mineral oil to enhance the glide performance is described in US2008/0060201. However the art also discloses a reduction of the swelling and solubility of the polymer matrix. The ability of the polymer matrix to swell in contact with water is however believed to be the key mechanism by which the lubrication benefit is delivered to the skin. Hence this is not desirable, as it will negatively impact the overall performance.

Another limitation of such shaving aids is related to the manufacturing process which typically involves an injection molding or extrusion process step. These processes require elevated temperatures in order to melt all the component materials and then subsequently mix them together and then injection mold or extrude. Consequently, the manufacture of such shaving aids is limited to low levels of additives and or materials which are not degraded by such process conditions. Nevertheless, the presence of even low levels of such additives in the manufacturing process can result in barrel slip and conveying inconsistencies, which is also undesirable.

Alternatively, GB2138438 describes a solid shaving composition comprising polyethylene oxide and various components which is wet granulated with 20%-80% isopropyl alcohol, a hydrophilic compound, followed by drying and blending of additional components. The mixture is subsequently compressed to a desired form. The use of such high levels of hydrophilic wet granulating agents may impact the resultant lubrication performance and reduce the amount of actives and or polymer matrix provided. Moreover, granulation will result in increased particle size which may affect the flexibility of the tablet size and performance of the resultant composition. Wet granulation may improve tablet density and alters particle flowability but results in a larger average particle and consequently may require a larger container for attachment to the razor cartridge.

Another alternative approach described in the art is the use of encapsulates to protect temperature sensitive materials, such as described in EP 2286787. However encapsulation has limited application in shaving aids, as the encapsulate must provide the dual function of encapsulating and protecting the sensitive material from temperature and process degradation, whilst still ensuring its controlled release without impacting the overall performance. Moreover such systems typically only allow low loading of hydrophobic actives and are also expensive.

Consequently, there is still a need to provide a lubricating member for razor cartridges comprising a water soluble polymer exhibiting improved lubricating properties which can be readily manufactured without impacting performance.

### SUMMARY OF THE INVENTION

One aspect of the invention relates to a solid compressed lubricating member for a razor cartridge comprising:
a) from 10% to 90% by weight of a particulate material comprising a water soluble polymer and
b) from 1% to 40% by weight of a silicone polymer or mixtures thereof;
wherein at least a portion of said water soluble polymer is spray coated with said silicone polymer compound.

Another aspect of the invention relates to a method of manufacturing said lubricating member comprising the steps of
1) Providing a particulate of said water soluble polymer,
2) spray coating said silicone polymer onto said water soluble polymer particulates and
3) applying pressure onto said spray coated particulates to form a tablet

Preferably the method further comprises the steps of:
1) Forming a liquid of said silicone polymer prior to said spray coating step,
2) Filling a pre-form with said particulates prior to the application of pressure and
3) Removing said compressed form from said pre-form.

### BRIEF DESCRIPTION OF THE DRAWINGS

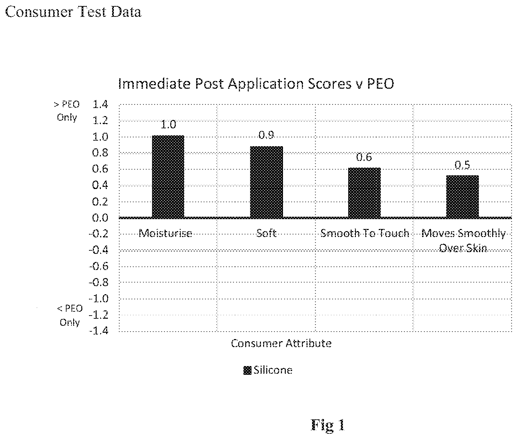

Figure 1 is a graph depicting the consumer test data results of a lubricating member according to the invention, versus a comparative lubricating member without a silicone polymer compound.

### DETAILED DESCRIPTION OF THE INVENTION

### Water soluble polymer

According to the invention the solid compressed lubricating member comprises from 10% to 90% by weight of a particulate material(s) comprising a water soluble polymer or mixture thereof. The lubricating member may comprise at least about 50%, preferably at least about 60%, more preferably up to about 90%, by weight of the water soluble polymer. The particulate material is solid at 25°C and preferably has a melting point of 30°C or more.

Examples of suitable water soluble polymers include polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, and mixtures thereof. In some embodiments, said water soluble polymer is selected from the group consisting of polyethylene oxide, polyethylene glycol, and mixtures thereof.

The preferred water soluble polymers are the polyethylene oxides generally known as POLYOX (available from Union Carbide Corporation) or ALKOX (available from Meisei Chemical Works, Kyoto, Japan). The water soluble polymer, (especially these polyethylene oxides), may have average molecular weights of at least about 20,000, preferably at least about 50,000, more preferably at least about 100,000 or from about 100,000 to about 6 million, preferably about 300,000 to about 5 million. A particularly preferred polyethylene oxide comprises a blend of about 40% to 80% of polyethylene oxide having an average mol.wt. of about 5 million (e.g. POLYOX COAGULANT) and about 60% to 20% of polyethylene oxide having an average mol.wt. of about 300,000 (e.g. POLYOX WSR-N-750). The polyethylene oxide blend may also advantageously contain up to about 10% (for example about 5%) by weight of a low mol.wt. (i.e. MW<10,000) polyethylene glycol such as PEG-100.

### Silicone polymer

According to the invention the lubricating member further comprises from about 1% to about 40%, preferably from about 12% to about 40%, more preferably from about 12% to about 30%, even more preferably 15% to 25% by weight of a silicone polymer or mixtures thereof. The silicone polymer is preferably provided in a liquid form so that it can be spray coated onto the particulate material as described hereinafter. Preferably the silicone polymer is liquid at 25°C. Suitable silicone polymers include oils and or waxes and mixtures thereof. The silicone polymer can provide a number of in use benefits such as lubrication and skin feel.

In one preferred embodiment, the silicone polymer or mixtures thereof is a substance(s) that is very slightly soluble, preferably practically insoluble in water according to the United States Pharmacopeia (USP) definition in 31/NF 26 Vol. 2 General Notices, Page Xvii. According to that definition, very slightly soluble means that 1000 to 10000 parts of water are needed to dissolve 1 part solute and practically insoluble means that more than 10,000 parts of water are needed to dissolve 1 part solute respectively.

In another preferred embodiment the silicone polymer or mixture thereof may have a melting point of 45°C or less, preferably 40°C or less, even more preferably 30°C or less, most preferably 25°C or less, such that it can be provided in a liquid form for spray coating. The melting point is determined according to ASTM D5440-93. Preferably the silicone polymer is liquid at 25°C. In another preferred embodiment the silicone polymer or mixtures thereof may be very slightly soluble and have a melting point of 45°C or less as defined above.

Non-limiting examples of suitable silicone oils include dimethicones (including partial esters of dimethicones and fatty acids derived from natural/synthetic oils), cyclomethicones, phenylated silicones, phenyl trimethicones, trimethyl pentaphenyl trisiloxane and mixtures thereof.

Non-limiting examples of commercially available silicone oils include Dow Corning 200 fluid, Dow Corning 244, Dow Corning 245, Dow Corning 344, and Dow Corning 345, (commercially available from Dow Corning Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.), the Viscasil series (sold by General Electric Company), SF 1075 methyl-phenyl fluid (sold by General Electric Company) and 556 Cosmetic Grade Fluid (sold by Dow Corning Corp.), SF1066 organosilicone surfactant (sold by General Electric Company) and Silshine 151 (sold by Momentive) and PH1555 and PH1560 (sold by Dow Corning).

Suitable silicone waxes include, but are not limited to, any silicone that is solid at 25°C; very slightly soluble in water, preferably practically insoluble in water according to the United States' Pharmacopeia (USP) definition in 31/NF 26 Vol. 2 General Notices, Page Xvii. According to that definition, means that 1000 to 10000 parts of water are needed to dissolve 1 part solute and means that more than 10,000 parts of water are needed to dissolve 1 part solute respectively); has an onset temperature measured according to the Differential Scanning Calorimetry (DSC) melting Method, defined herein below, which is 10°C or greater; and comprises silicones or mixtures thereof.

Non-limiting examples of suitable silicone waxes include DC2503 Cosmetic Wax, DC580 Wax, DC AMS-C30 Cosmetic Wax, C30-45 Alkyl Methicone, DC Silkywax 10, Hexamethyldisiloxane, DC ST-Wax 30, C30-45 Alkyldimethylsilyl Polypropylsilsesquioxane, DC SW-8005 Resin Wax, C26 - 28 Alkyl Dimethicone, C26 - 28 Alkyl Methicone, Polyphenylsilsesquioxane and mixtures thereof.

Preferred silicone polymers may be selected from dimethicones, phenylated silicones and mixtures thereof.

Suitable silicone polymers for use herein may be selected such that they have a surface tension of 35mN/m or less, preferably 30 mN/m or less, more preferably 25mN/m or less. Surface tension is determined according to ASTM D1331-1.

### Hydrophobic compound

According to the invention, the lubricating member may further comprise a hydrophobic compound or mixtures thereof. In one embodiment the lubricating member comprises from about 1% to about 40%, preferably from about 5% to about 40%, more preferably from about 10% to about 40%, even preferably from about 12% to about 30% by weight of a hydrophobic compound and or mixtures thereof. The hydrophobic compound is preferably provided in a liquid form so that it can be spray coated onto the particulate material as described hereinafter in addition to or combined with the silicon polymer. Preferably the hydrophobic material is liquid at 25°C. Suitable hydrophobic compounds include natural oils and or waxes and or fats; synthetic waxes or oils; triglycerides; skin active agents, sensates, fragrance oils and mixtures thereof. The hydrophobic material can provide a number of in use benefits such as lubrication, skin feel and cooling sensation.

In one preferred embodiment, the hydrophobic compound or mixtures thereof is a substance(s) that is very slightly soluble, preferably practically insoluble in water according to the United States' Pharmacopeia (USP) definition in 31/NF 26 Vol. 2 General Notices, Page Xvii. According to that definition, very slightly soluble means that 1000 to 10000 parts of water are needed to dissolve 1 part solute and practically insoluble means that more than 10,000 parts of water are needed to dissolve 1 part solute respectively.

In another preferred embodiment the hydrophobic compound or mixture thereof may have a melting point of 45°C or less, preferably 40°C or less, even more preferably 30°C or less, most preferably 25°C or less, such that it can be provided in a liquid form for spray coating. The melting point is determined according to ASTM D5440-93 Preferably the hydrophobic material is liquid at 25°C. In another preferred embodiment the hydrophobic compound or mixture thereof may be very slightly soluble and have a melting point of 45°C or less as defined herein above.

Suitable hydrophobic compounds for use herein include for example natural oils, synthetic oils, or mixtures thereof. As used herein, the term "oil" includes, but is not limited to any nonaqueous substance that is very slightly soluble, preferably practically insoluble in water according to the United States' Pharmacopeia (USP) definition in 31/NF 26 Vol. 2 General Notices, Page Xvii. According to that definition, means that 1000 to 10000 parts of water are needed to dissolve 1 part solute and that more than 10,000 parts of water are needed to dissolve 1 part solute respectively and is liquid at 25°C.

The oil may be selected from natural oil, synthetic oil and mixtures thereof. Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, Caprylic/Capric/Linoleic Triglyceride, Caprylic/Capric/Myristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil, Carya IlIinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, lsatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil mineral oil and mixtures thereof.

Suitable synthetic oils include hydrocarbons, esters, alkanes, alkenes and mixtures thereof. Non-limiting examples include isopropyl palmitate, isopropyl stearate, isohexadecane, isododecane, polyglyceryl triisostearate and mixtures thereof. Preferred oils may be selected from capric and or caprylic triglycerides, grape seed oil, olive oil and mixtures thereof.

Suitable hydrophobic compounds also include fats and or waxes such as natural, synthetic and silicone waxes. As used herein, the term "wax" and "fat" include, but is not limited to, any hydrophobic material that is solid at 25°C; very slightly soluble in water, preferably practically insoluble in water according to the United States' Pharmacopeia (USP) definition in 31/NF 26 Vol. 2 General Notices, Page Xvii. According to that definition, means that 1000 to 10000 parts of water are needed to dissolve 1 part solute and means that more than 10,000 parts of water are needed to dissolve 1 part solute respectively; has an onset temperature measured according to the Differential Scanning Calorimetry (DSC) melting Method, defined herein below, which is 10°C or greater; and comprises lipids, silicones or mixtures thereof.

The wax may comprise natural wax, synthetic wax or mixtures thereof. Non-limiting examples of suitable natural waxes include Abies Alba Leaf Wax, Acacia Dealbata Leaf Wax, Acacia Farnesiana Flower Wax, Beeswax, Ceresin, Cetyl Esters, Cistus Labdaniferus Flower Wax, Aurantium Amara (Bitter Orange) Flower Wax, Aurantium Dulcis (Orange) Peel Wax, Copernicia Cerifera (Carnauba) Wax, Eclipta Prostrata Wax, Euphorbia Cerifera (Candelilla) Wax, Helichrysum Angustifolium Wax, Jasminum Officina le (Jasmine) Flower Wax, Jasminum Sambac (Jasmine) Flower Wax, Jojoba Esters, Jojoba Wax, Lanolin Wax, Lavandula Angustifolia (Lavender) Flower Wax, Lawsonia Inermis Wax, Mink Wax, Montan Acid Wax, Montan Wax, Myrica Cerifera (Bayberry) Fruit Wax, Ocimum Tenuiflorum Wax, Olive Wax, Oryza Sativa (Rice) Bran Wax, Ouricury Wax, Palm Kernel Wax, Persea Gratissima (Avocado) Wax, Pistacia Lentiscus Leaf Wax, Polianthes Tuberosa Flower Wax, Pyrus Malus (Apple) Peel Wax, Ribes Nigrum (Black Currant) Wax, Rosa Centifolia Flower Wax, Salvia Sclarea (Clary) Wax, Shellac Wax, Simmondsia Chinensis (Jojoba) Butter, Soft Olive Wax, Spent Grain Wax, Stipa Tenacissima Wax, Sunflower Seed Wax, Vegetable Wax, Vitis Vinifera (Grape) Leaf Wax, Petrolatum and mixtures thereof.

Non-limiting examples of suitable synthetic waxes include Hydrogenated Japan Wax, Hydrogenated Jojoba Oil, Hydrogenated Jojoba Wax, Hydrogenated Microcrystalline Wax, Hydrogenated Rice Bran Wax, Hydrolyzed Beeswax, Microcrystalline Wax, Oxidized Beeswax, Oxidized Microcrystalline Wax, Ozokerite, Paraffin, PEG-6 Beeswax, PEG-8 Beeswax, PE G-12 Beeswax, PEG-20 Beeswax, PEG-12 Carnauba, Potassium Oxidized Microcrystalline Wax, Sulfurized Jojoba Oil, Synthetic Beeswax, Synthetic Candelilla Wax, Synthetic Carnauba, Synthetic Japan Wax, Synthetic Jojoba Oil, Synthetic Wax and mixtures thereof.

The hydrophobic compound(s) may comprise one or more triglycerides, each triglyceride, having the following formula: 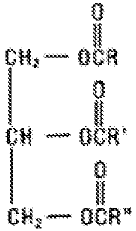 wherein R, R' and R" may be the same as or different from one or both of the others, wherein each of R, R' and R" is a fatty acid and wherein each triglyceride is solid at 25°C

Suitable oils from which triglycerides may be formed from include, but are not limited to, the oils listed herein. Suitable fatty acids for formation of triglycerides include, but are not limited to, Myristoleic acid, Palmitoleic acid, Sapienic acid, Oleic acid, Linoleic acid, α-Linolenic acid, Arachidonic acid, Eicosapentaenoic acid, Docosahexaenoic acid, Lauric acid (C₁₂), Myristic acid (C₁₄), Palmitic acid (C₁₆), Stearic acid (C₁₈), Arachidic acid (C₂₀) and mixtures thereof.

Specific sources of triglycerides suitable for inclusion herein include Butter, Shea Butter, Butyrospermum Parkii, Lipex Shea, Theobroma Cacao (Cocoa) Seed Butter, Cocoa Butter, Hydrogenated Shea Butter, Hydrogenated Cocoa Butter, Irvingia Gabonensis Kernel Butter, Tallow, Lard, Mangifera Indica (Mango) Seed Butter, Kokum Butter and mixtures thereof. Particularly preferred are shea butter, cocoa butter and mixtures thereof. The triglyceride(s) may fall under the definition of "wax" (or "fat") or "oil" as used herein and, in such a case, should be included as a wax (or fat) or oil for the purposes of determining the proportions of wax or oil.

The hydrophobic material may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils; peppermint oil, Iso E Super [(1-(1,2,3,4,5,6,7,8-octahydro-2,3,8,8-tetramethyl-2-naphthalenyl)ethanone]; and mixtures thereof. Particularly preferred are lanolin, essential oils, peppermint oil and mixtures thereof. These materials may fall under the definition of "wax" or "oil" as used herein and, in such a case, should be included as a wax or oil for the purposes of determining the proportions of wax or oil.

In some embodiments, the hydrophobic compound comprises one or more sensates. A large number of coolant compounds of natural or synthetic origin are known. The most well know is peppermint oil. Among synthetic coolants, many are derivatives of or are structurally related to menthol, i.e., containing the cyclohexane moiety, and derivatized with functional groups including carboxamide, ketal, ester, ether and alcohol. Non-limiting examples include methyl emthylamido oxalate, (under the tradename Frescolat X-cool available from Symrise), menthyl lactate (such as Frescolate ML Natural available from Symrise), and Menthyl Pyrrolidone Carboxylate also known as Menthyl PCA (under the tradename Questices available from Givaudan).

The lubricating member may comprise more than one sensate. In this case, at least one sensate is a hydrophobic compound which in liquid form is used to dissolve one or more additional sensates. In particular, peppermint oil can be used to dissolve a variety of other sensates which include N substituted methanecarboxamides having the formula: 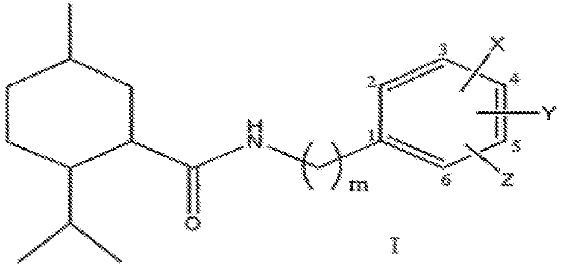 in which m is 0 or 1, Y and Z are selected independently from the group consisting of H. OH, C1-C4 straight or branched alkyl, or, a C1-C4 straight or branched alkoxy, X is (CH2)n-R, where n is 0 or 1 and R is a group with non-bonding electrons, with the provisos that: (a) when Y and Z are H. X is not F, OH, MeO or NO2 in the 4-position and is not OH in the 2 or 6-position, and (b) when Y or Z is H then X, Y and Z are such that (i) the groups in the 3- and 4-positions are not both OMe, (ii) the groups in the 4- and 5-positions are not both OMe, (iii) the groups in 3-and 5- positions are not OMe if the group in the 4-position is OH, and (iv) the groups in the 3-and 5-positions are not OH if the group in the 4- position is methyl.

The preferred compounds are those in which X is in the 4-position. The most preferred compounds are when X is in the 4-position and Y and Z are H, OH, Me or OMe. Preferred groups with non-bonding electrons are halogens, OH, OMe, NO2, CN, Ac, SO2NH2, CHO, CO2H and C1-C4 alkyl carboxylates such as CO2Et.

One specific example of a suitable N-substituted menthanecarboxamide is N-[4-(cyanomethyl)phenyl]-(1R,2S,5R)-2-isopropyl-5-methylcyclohexanecarboxamide of Formula II which is available from Givaudan as a solution concentration of 8% in peppermint oil.

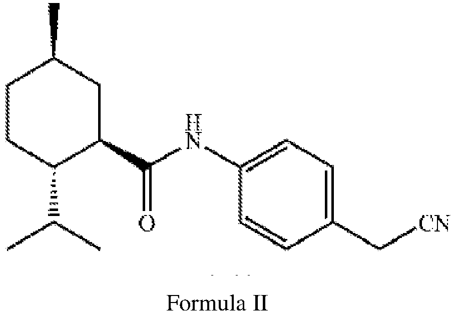

This material is also commonly referred to as N-para-benzene acetonitrile menthane carboxamide. *See e.g.* Research Disclosure RD 522003 (Givaudan), U.S. Patent Pub. Nos. 2009/0311206 and 2009/0306152, both assigned to Beiersdorf, 2006/0276667, 2010/0086498, and U.S. Patent 7,414,152.

Other sensates may comprise a menthane carboxylic acid-N-(4-methoxyphenyl)-amide of formula (A):

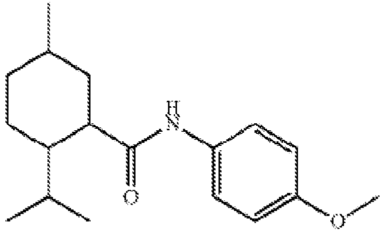

Or wherein said menthane carboxylic acid-N-(4-methoxyphenyl)-amide has the formula (B):

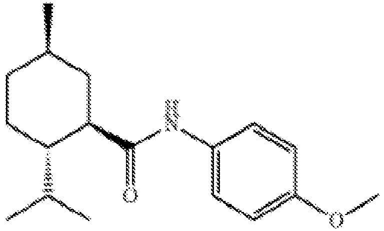

Non-limiting examples of such menthane carboxylic acid-N-(4-methoxyphenyl)-amides are disclosed in U.S. Patent Pub. 2011/0081303, and 2010/0086498. This material is also described under CAS#68489-09-8, may also be named (1*R**,2*S**)-*N*-(4-Methoxyphenyl)-5-methyl-2-(1-methylethyl)cyclohexanecarboxam-ide and is commercially available as SC1, WS-12 or Frescolat MMC by Symrise, Inc.

A suitable hydrophobic compound may also be a hydrophobic liquid formed by mixing two solids that form a eutectic mixture. Non-limiting examples include the mixture of menthol and methyl lactate, such as Frescolat Plus available from Symrise, and the mixture of N-ethyle p-menthane-3-carboxamide (also known as WS-3) and N-2,3-trimethyl-2-ispropyl butanamide (also known as WS-23), such as Ice 1500 available from Qaroma.

Preferred hydrophobic compounds include capric and or caprylic triglycerides, grape seed oil, olive oil, shea butter, cocoa butter, lanolin, essential oils, peppermint oil, isoheaxdecane, petrolatum and mixtures thereof. More preferred is petrolatum.

### Optional Ingredients

The lubricating material may further comprise additional components, preferably components which are solid, dry components and preferably suitable for particulate compression. The term dry components refers to components comprising less than 10%, preferably less than 1% by weight of water.

Suitable additional components include cationic polymers. Suitable cationic polymers are, for example, cationic cellulose derivatives, for example a quaternized hydroxymethyl cellulose obtainable under the name Polymer JR 400® from Amerchol, cationic starches, copolymers of diallylammonium salts and acrylamides, quaternized vinylpyrrolidone/vinyl imidazole polymers, for example Luviquat® (BASF), condensation products of polyglycols and amines, quaternized collagen polypeptides, for example lauryldimonium hydroxypropyl hydrolyzed collagen (Lamequat®L/Grünau), quaternised wheat polypeptides, polyethyleneimine, cationic silicone polymers, for example amidomethicones, copolymers of adipic acid and Dimethyl-aminohydroxypropyldiethylenetriamine (Cartaretin®/Clariant), copolymers of acrylic acid with dimethyldiallylammonium chloride (Merquat® 550/Chemviron), polyaminopolyamides, as described, for example, in FR-A-2 252840, and the cross-linked water-soluble polymers thereof, cationic chitin derivatives, for example of quaternised chitosan, optionally distributed as microcrystals; condensation products of dihaloalkyls, for example dibromobutane, with bisdialkylamines, for example bisdimethylamino-1,3-propane, cationic guar gum, for example Jaguar® C-17, Jaguar® C-16 from Celanese, quaternised ammonium salt polymers, for example Mirapol® A-15, Mirapol® AD-1, Mirapol® AZ-1 from Miranol. As anionic, zwitterionic, amphoteric and non-ionic polymers there come into consideration, for example, vinyl acetate/crotonic acid copolymers, vinylpyrrolidone/vinyl acrylate copolymers, vinyl acetate/ butyl maleate/isobornyl acrylate copolymers, methyl vinyl ether/maleic anhydride copolymers and esters thereof, uncrosslinked polyacrylic acids and polyacrylic acids cross linked with polyols, acrylamidopropyltrimethylammonium chloride/acrylate copolymers, octyl acrylamide/methyl methacrylate/tert-butylaminoethyl methacrylate/2-hydroxypropyl methacrylate copolymers, polyvinylpyrrolidone, vinylpyrrolidone/vinyl acetate copolymers, vinylpyrrolidone/ dimethyl-aminoethyl methacrylate/vinyl caprolactam terpolymers and also optionally derivatised cellulose ethers and silicones. Furthermore the polymers as described in EP 1093796 (pages 3 - 8, paragraphs 17 - 68) may also be used.

The lubricating material may further comprise a copolymer of polyethylene oxide (PEO) and polypropylene oxide (PPO). The PEO/PPO copolymer may have an average molecular weight of at least 5,000, preferably in the range of from 10,000 to 20,000, more preferably from 11,000 to 15,000, even more preferably from 12,000 to 13,000 and even more preferably still from 12,250 to 12,750 Without wishing to be bound by theory, the inclusion of a PEO/PPO copolymer of sufficient molecular weight is thought to further improve the lubrication properties of the lubricating member in aqueous conditions, especially in combination with a further water soluble polymer (particularly polyethylene oxide), and thus prevent an undesirable feeling in use.

The PEO/PPO copolymer may advantageously be a block copolymer, for example a di-block, tri-block, multi-block, radial-block or random-block copolymer. Preferably, the PEO/PPO copolymer is a tri-block copolymer, more preferably a tri-block copolymer having the sequence: PEO-PPO-PEO, the later commercially available under tradenames such as Pluracare from BASF and Pluronic from Sigma-Aldrich.

The PEO/PPO copolymer may have a weight ratio of PEO to PPO (i.e. of ethylene oxide repeat units to propylene oxide repeat units), of from 1000:1 to 1:1000 or from 100:1 to 1:100.

Advantageously, the weight ratio is selected to improve the solubility properties of the PEO/PPO copolymer in a system comprising a water-soluble polymer (especially polyethylene oxide) and water, and so may be from 10:1 to 1:10, preferably from 1:1 to 1:7 (or any ratio in which the weight of PPO is greater than or equal to the weight of PEO), more preferably from 1:2 to 1:5, even more preferably from 1:2.5 to 1:4 and even more preferably still from 1:2.5 to 1:3. The PEO/PPO copolymer may have an HLB of from 0 to 50, advantageously from 1 to 30, preferably from 5-25, more preferably from 10-25, even more preferably from 17-24 and even more preferably still from 18-23.

The PEO/PPO copolymer is typically present at an amount of from 0.01% to 50%, preferably from 0.01% to 50%, more preferably from 2% to 40%, even more preferably from 3% to 25%, even more preferably still from 4% to 20% and most preferably from 5% to 10% by weight of the lubricating material or by weight of the lubricating member.

The lubricating member may also further comprise a water-insoluble material such as hydrophobic binders. Such components may enhance the life of the lubricating material by reducing its tendency to be mechanically eroded. Advantageously, the hydrophobic binder is solid at standard temperature and pressure. Suitable hydrophobic binders include divalent metal cation stearate, preferably magnesium stearate, calcium stearate, zinc stearate, or mixtures thereof, more preferably magnesium separate; ethyl cellulose; polycaprolactone; polyethylene; polypropylene; polystyrene; butadiene-styrene copolymer (e.g. medium and high impact polystyrene); polyacetal; acrylonitrilebutadiene-styrene copolymer; ethylene vinyl acetate copolymer and blends such as polypropylene/polystyrene blend; and mixtures thereof. The lubricating material may comprise from 1 to 20%, more preferably from 5 to 15% by weight of hydrophobic binder. The hydrophobic binder may fall under the definition of hydrophobic compound as used herein and in such a case should be included for purposes of determining the amount by weight of the hydrophobic compound or mixture.

### Further Optional Ingredients

In some embodiments, the lubricating material may comprise any other ingredients commonly found in commercially available shaving aid members. The lubricating member may therefore contain other conventional shaving aid ingredients, preferably suitable for particulate compression such as low molecular weight water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, skin feel / care actives, surfactants, soaps (including interrupted soaps), antioxidants, preservatives, emollients, beard softeners, astringents, medicinal agents, plasticizers, additional lubricants, depilatories/keratolytic materials, tackifiers, skin-soothing agents, fragrances, compatibilisers, anti-inflammatory agents, antipruritic/counterirritant materials etc and mixture thereof. These ingredients may fall under the definition of hydrophobic compounds as used herein and should be included as such in determining the amount of hydrophobic compounds.

### Method of manufacture/processing

The lubricating member may be manufactured according to the steps described below whereby the water soluble polymer and other solid dry components are provided as particulates and mixed. The silicone polymer is, if necessary, liquefied and then spray coated onto at least a portion of the water soluble polymer particulate and other dry components if present. Preferably at least 95%, more preferably at least 98% and even more preferably substantially 100% of the water soluble polymer and preferably any other dry particulate material components present are spray coated with the silicone polymer. Hydrophobic compounds if present may also be spray coated as a mixture with the silicone polymer or in a separate spray coating step.

Any known method of spray coating may be used herein. Preferably the silicone polymer (and optional hydrophobic compound) is sprayed at a droplet size of about 0.1 to 250 µm, more preferably 1 to 10µm. Typically the flow rate is less than 1GPM. Typically the droplet size is selected with regard to the particulate material size and is preferably less than 10% of the size of the particles. Whilst not being bound by theory, spray coating the silicone polymer enables a uniform distribution of the material onto the water soluble polymer and other dry components, if present and consequently uniform delivery onto the skin in use from the resultant lubricating member. Furthermore, the use of compression compaction to form the lubricating member from the spray coated particulates enables the use of a large variety of silicone polymers or mixtures thereof, and optional hydrophobic compounds, providing multiple in use consumer benefits.

The term compression and or compression molding or compression compaction as used herein refers to a process by which the bulk density of a particulate or powder is reduced to form a solid tablet by the application of pressure. Typically, this is performed without the application of external shear force or heat. Preferably the compression compaction is conducted below the melting point of at least one, preferably all the particulate components, preferably at an ambient temperature of 25°C. As such the particulates retain their integrity after the compression process and are typically visible by the naked eye after the compression process is completed.

In certain embodiments, additional energy sources such as heat may be applied during or post compression to increase inter-particulate bonding and increase the rigidity of the resulting lubricating member but which preferably does not resulting in any substantial melting of the particulate material. Preferably however the method of the present invention does not require an extrusion or injection molding step or the application of energy sources such as heat.

The lubricating member is provided in the form of a compressed powder. As used herein the term "compressed powder" refers to a particulated material which is subsequently compressed, preferably using cold compression techniques. Preferably the particulates have an average particle size distribution of from about 50 to about 1250 microns and preferably from about 300 to about 1250 microns, more preferably about 1000 microns. Alternatively the particulate size is such that 90% of particles pass through a 20 mesh screen; i.e. 90% of particles are less than 841micron in diameter. The lubricating member is compressed preferably directly into a preform or container with a compression force of typically greater than 1KN. This may be achieved using any method and equipment known in the art such as a die press. The bulk density of the particulate material prior to compression is typically about 300 to about 600 kg/m³ and increases to about 1000 to about 1200kg/m³ following compression. Bulk density thus may be increased by about 200% to about 400% after the compression. Whilst not bound by theory, it has been found that the use of particulate compression manufacturing, preferably cold particulate compression (i.e. at 25°C or less) to form the lubricating member enables highly lubricous components to be incorporated therein whilst not negatively impacting the water solubility and swelling performance of the water soluble polymer. This also allows flexibility in the size of the resulting lubricating member to be used for multiple razor cartridges whilst enabling the effective delivery of the silicone polymer to the skin of the consumer in use.

According to the invention the lubricating member may be manufactured according to the following steps:
1. Optionally forming a particulate of said water soluble polymer and other solid dry components,
2. Providing a particulate of said water soluble polymer, and other solid dry components, preferably at 25°C, at the desired average particle size,
3. Mixing the particulate dry components using a suitable powder mixing device such as a V Blender (nominal Shell RPM 25 RPM) until visibly uniform and free from agglomerates.
4. Providing the silicone polymer component in a liquid state, for example if necessary by the application of heat to reach the melting point of the silicone polymer and maintaining the temperature in order to spray coat silicone polymer in the liquid state.
5. Spray coating the silicone polymer (and optionally other hydrophobic compounds) onto the mixed dry components (i.e. water soluble polymer particulates and other dry particulate components), preferably whilst mixing the dry components.
6. Continuing mixing until uniform (for example by use of oil soluble dye and light microscopy), preferably using high shear (such as V blender at nominal shell of 3600RPM).
7. Optionally passing the mixture through a granulation screen such as Fruend Vector TF Mini roller at 2Ton roller pressure at 3.1 RPM and screw feed at 12.7 RPM
8. Compression compacting the mixture for example by die compression whereby mixture is added to desired die/reservoir perform and a die punch applied at a pressure of 0.5-10kN at 25°C.
9. Removing the punch and ejecting the tablet of compressed particulate mixture from the die in desired end size and shape tablet.

Optionally step 8 may include elevating the temperature of the mixture by applying a heat source so that the mixture has a temperature of about 75°C before applying the die punch as described above. The die is then cooled to about 25°C prior to removing the punch and ejecting the tablet as described above.

### Hair Removal Head

According to some embodiments of the invention, the lubricating member finds particular application for hair removal devices. Hair removal devices generally comprise a hair removal head and a handle or grip portion, upon which the hair removal head is mounted. The hair removal device can be manual or power driven and can be used for wet and/or dry application. The hair removal head can include a wide scraping surface such as where the hair removal device is used with a depilatory, or be a razor cartridge or foil where the device is a shaving razor. The hair removal head may be replaceable and/or pivotally connected to a cartridge connecting structure and in turn or independently (e.g. permanently fixed) to a handle. In some embodiments, the cartridge connecting structure includes at least one arm to releasably engage the hair removal head.

The hair removal head typically comprises one or more elongated edges usually positioned between a first and second end, said one or more elongated edges comprising a tip extending towards said first end. Where the hair removal head is a razor cartridge the one or more elongated edges can include blades. For example, U.S. Patent 7,168,173 generally describes a Fusion® razor that is commercially available from The Gillette Company and which includes a razor cartridge with multiple blades. Additionally, the razor cartridge may include a guard as well as a skin engaging member. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the lubricating member can be used with any currently marketed system or disposable razor, including those having 2, 3, 4 or 5 blades. In such a case, the hair removal device is a razor, the hair removal head is a razor cartridge and the one or more elongated edges are blades. Another example of a hair removal device is a scraping tool for use with a hair removal composition, i.e. a depilatory.

In some embodiments, said at least one lubricating member is located on the portion of the cartridge that contacts skin during the hair removal process, forward and/or aft of the blades. A feature "forward" of the one or more elongated edges, for example, is positioned so that the surface to be treated with by the hair removal device encounters the feature before it encounters the elongated edges. A feature "aft" of the elongated edge is positioned so that the surface to be treated by the hair removal device encounters the feature after it encounters the elongated edges. Where more than one lubricating member is provided on the hair removal device, they can be the same (identical) or different, in terms of physical shape/structure and/or chemical composition, and one or more of them may comprise the spray coated particulate.

In some particular embodiments, a plurality (e.g. 2, a first and second) of lubricating members may be provided on the hair removal head, with the first skin engaging member comprising the same composition or different. These lubricating members may be placed collectively (for example adjacent to one another) ahead of or behind the elongated edges (e.g. blades on a razor cartridge), including side by side, or separately with one ahead of the elongated edges and the other behind.

The lubricating member may be free standing utilizing a suitable attachment means such as adhesive or may be contained at least partially within a container.

The container typically has a base and at least one side wall extending vertically preferably perpendicular from said base and a skin contacting surface. In a preferred embodiment said container comprises a base and at least 2 side walls, more preferably at least 4 side walls, preferably said walls completely enclosing the base. Typically, each pair of walls are substantially parallel and preferably one pair of walls is substantially parallel to the at least two blades. Alternatively, the base may be enclosed by a one piece single wall. The container may form any shape including substantially rectangular, or oval. The container typically has a front wall adjacent the blades and a rear wall, preferably substantially parallel thereto and furthest from said blades.

The container is preferably further provided with at least one dispensing orifice for dispensing the lubricating member onto the skin during use. In one embodiment the container is provided with a top extending substantially perpendicular from the side wall (s). The container would, in such an embodiment, typically have a receiving region for receiving the lubricating member. The top may be substantially parallel to the base or it may be provided at an angle such that the distance of the top from the blade plane increases or decreases as the distance of the container from the blades increases. In one embodiment the height of the top of the container increases in distance from the blade plane as the container distance from the blades increases. In an alternative embodiment the height of the top of the container decreases in distance from the blade plane as the container distance from the blade increases.

The orifice may be of any shape and may, for example, have a cross sectional area of from about 0.00324 to about 1.613 cm2. Small orifices can also be provided with cross sectional area of from about 0.0324 to about 0.324 cm², or from about 0.0645 to about 0.16135 cm². Larger orifices can have cross sectional areas of from about 0.324 to about 1.613 cm², or from about 0.645 to about 1.29 cm². The container may comprise a single orifice or multiple orifices which may be large and or small. In one embodiment the container comprises at least two orifices. Combinations of small and large orifices can also be provided on the same skin engaging member, or on separate members on the same cartridge, depending on the desired dispense rate and amount of exposure of the lubricating material to water. In one embodiment the top of the container is provided with one preferably two orifices, more preferably two substantially identical orifices adjacent one another.

The skin engaging surface of the container which has a surface area, while the at least one orifice (i.e. the sum for all orifices if a plurality are present) has a cross sectional area such that the surface area and cross sectional area are in a ratio of from about 50:1 to about 1:1, or about 25:1 to about 2:1, or about 10:1 to about 3:1.

In some embodiments, at least a portion of said container is not linear for example angled or curvilinear. Curvilinear as defined herein means that at least a portion is curved such that it does not form a straight line. Where at least two containers are provided, they can also be positioned relative to one another such that they do not form a straight line. Alternatively, the curved or angled nature is such that it forms at least a partial ring. A partial ring, as defined herein, means that the structure has at least two curved or angled sections which are concave to form an inner region. The partial ring can also include a curved or angled portion which is positioned convex to said inner region. One or more of said containers may also be positioned relative to one another to form a full ring.

The container can be formed of a variety of materials. The container may, preferably be for example, provided from a non-water soluble material such that it does not degrade or dissolve during normal use.

The container typically has sufficient mechanical strength and rigidity to provide adequate mechanical strength to the entire skin engaging member, both as initially produced and after a significant amount of lubricating material has leached out of the container. Alternatively or in addition a further reinforcing member may also be utilized. In some embodiments, the container comprises a base and one or more side walls, forming a receiving region, or channel, onto or into which the lubricating material is placed.

The side walls may or may not be the same height (as measured extending away from the base of the container). At least one of the side walls can have a height of about 0.1 cm to about 1 cm, preferably from about 0.2 cm to about 0.4 cm. The pair of side walls can be biased away from each other as the walls extend away from said base, or they can be biased towards each other. At least one wall extends vertically from the base and is preferably perpendicular to the blade plane (P). One or both ends of the container can be enclosed, e.g. as described in US 7,581,318. The term maximum height of at least one wall as used herein refers to the first front wall preferably substantially parallel to the at least two blades and closest thereto or it refers to the rear wall farthest from said at least two blades. In one embodiment the said at least one wall is closest to said at least two blades. In an alternative embodiment the at least one wall is farthest from said at least two walls. In one embodiment, the ratio of the height of the front wall to the rear wall is from 5:1 to 1:5, more preferably from 2:1 to 1:2 and more preferably the height of the front wall is greater than the rear wall. The walls have a thickness of from 0.1cm to 1.0cm, preferably from 0.3 to 0.5cm.

The container may be made of a water-insoluble polymer, particularly a thermoplastic resin. Thermoplastic resins are those materials which can be extruded or molded into a shape and are resilient under normal environmental conditions such as contact with water, even up to normal household hot water temperatures (for example up to 125 °C); normal wear and tear by consumers during use; device assembly and shipping, etc. Thermoplastic resins suitable for use in the carrier include polystyrene, high impact polystyrene (polystyrene-butadiene), polypropylene, filled polypropylene, polyethylene, nylon ethylene vinyl acetate, and blends such as 70% nylon/30% polyethylene oxide, 60% polystyrene/40% polyethylene oxide butadiene styrene copolymer, polyacetal, acrylonitrile-butadiene styrene copolymer, and mixtures thereof. The preferred resins are high impact polystyrene, polystyrene, ethylene vinyl acetate (EVA), and mixtures thereof.

In some embodiments, the cartridge comprises a guard comprising at least one elongated flexible protrusion to engage a user's skin. The at least one flexible protrusion may comprise flexible fins generally parallel to said one or more elongated edges. Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower surface). In some embodiments, said lubricating member is positioned on the cartridge aft of the guard and forward of said elongated edge. In another embodiment, the lubricating member is positioned on the cartridge forward of the guard. This embodiment can be particularly useful to deliver the lubricating member prior to contact with the guard.

**Exemplified compositions**

| **Ingredient (weight %)** | **Comparative 1** | **Inventive 1** |
|---|---|---|
| Polyethylene oxide (Polyox N12K); (Dow Chemicals) | 70 | 50 |
| Copolymer of Polyethylene Oxide and Polypropylene Oxide (Pluronics F127 (BASF) | 20 | 20 |
| Guar hydroxyl propyltrimonium chloride (Nhance 3196 Ashland) | 10 | 10 |
| Dimethicone (DC200 350 cst Dow Corning) | - | 20 |

### Preparation:

1. Weigh materials into separate weigh boats.
2. Pre mix the powder materials together in a turbula shaker mixer T2F (drive belt positioned on smallest pulley) for 1min.
3. If needed, transfer powder blend in Lillo Due pasta mixer and start mixing at set speed.
4. Add the silicone polymer (premelt if needed) in a steady feed until all the liquid is incorporated into the powder.
5. Take the resulting powder blend and load it into a compression tool with a cavity size of 31.92 (length), 3.27 (width) and 1mm (depth).
6. Use the die punch to compress the powder at a force of 1 tonne for 15sec.
7. Remove the resulting tablet from the die and attach to a razor cartridge or use for analysis.

### Consumer test data

From the data shown in Figure 1, it can be seen that the inventive lubricating members provide consumer noticeable improved performance with regard to moisturisation, softness and glide verses comparative lubricating member without a silicone polymer compound.

### Consumer test method

10 UK male panellists were recruited for the testing. Each panellist was asked to shave with a commercially available Gillette Proglide Power cartridge using a Proglide manual handle using a Gillette Series sensitive gel. After shaving was completed the panellists were asked to pat dry the skin with a towel, but not to use any post shave products. A timer is then started for 2 minutes.

The comparative product and test product (lubricating member as described above adhered to a rectangular support which is attached to a Gillette MACH 3 handle) are provided to each panellist. The panellist is asked to thoroughly wet the product by running under the water for 1 minute. After 2 minutes, the panellist is asked to splash one side of their face once with water (as indicated). The panellist is then asked to use the product to take 3 downward non-overlapping strokes across one side of the cheek area of the face. The panellist is then asked to Re-wet the product and take another 3 downward non-overlapping strokes over the area of their face. This step is then repeated until a total of 9 downward strokes have been taken. The panellists are then asked to splash their face with water and pat dry their face with a paper towel (NO RUBBING). The panellists are then requested to wait 2 minutes post rinsing before touching their face.

Panellists are asked to complete questionnaire during the application and post-rinse. A 7 point dipolar scale is used, "Is" to "Is not".

### Swell Test Data

The swelling performance of the inventive 1 composition verses a comparative 1 control in the absence of the silicone polymer as detailed above, shows that the swell performance is not affected by the presence of the silicone polymer.

| | Time period: 120sec | |
|---|---|---|
| | Average | Std dev |
| Inventive example 1 | 319.12 | 80.61 |
| Comparative example 1 | 357.83 | 39.16 |

### Swell Test Method

1. Place a rectangular tablet of the required formulation into a small transparent receptacle. The receptacle should be sized so that the ends of the tablet touch the walls of the receptacle thus holding the tablet in place.
2. The receptacle is placed under a stereoscope (Olympus SZX7) on top of a piece of black card.
3. Set the magnification of the stereoscope to 3.2 and then focus to provide a clear image of the tablet.
4. Add 1ml of water dyed with 0.1%w/w of D&C red 28 on 1 side of the tablet.
5. The timer is started and photos are taken at t=0 seconds and then the following increments 5, 10, 30, 60, 90m 120 , 150 and 180 seconds using Pixera Pro 150ES software.
6. Repeat procedure for a minimum of 3 samples.
7. The height of the swollen gel is analysed at least 10 points across the length of the tablet using the Image Pro MC software
8. The data is then exported into EXCEL and swollen gel length is calculated for each time point by averaging the measurements for each tablet and between tablets. Statistical analysis is performed by calculating standard deviation.

### Differential Scanning Calorimetry (DSC) Melting Method

This method is the American Oil Chemists' Society Method Cj 1-94, as reapproved in 2009 and it determines the "onset temperature" (that is the temperature of onset of melting) of oils and fats by differential scanning calorimetry (DSC).

### Apparatus

1. Aluminium capsules.
2. DSC instrument, capable of holding temperature at -60°C and achieving a temperature of 80°C.

### Reagents

1. Indium, powder-60 mesh, 99.999%, such as Aldrich Chemical Co., Milwaukee, WI 53233, or equivalent.
2. *n*-Decane, 99+%, such as Aldrich Chemical Co., Milwaukee, WI 53233, or equivalent.
3. Methyl stearate, 99%, such as Aldrich Chemical Co., Milwaukee, WI 53233, or equivalent.

### Procedure

1. Standardization of equipment-Proceed with the normal standardization using both indium and *n*-decane as reference standards. Follow instrument manual for adjustment to lock onto these two reference points and flatten the baseline slope as much as possible when empty pans are analyzed. Analyze the secondary standard (methyl stearate). Weigh 5 mg of the standard into the same kind of pan which will be used for the test portion (if hermetically sealed, it may be reused at a later date). Use the method sequence in Procedure, 2-7 to obtain the melting point onset (because of the high purity, only a 2 min hold is necessary for the standard after crystallization). Be certain that the heating rate during the definitive heating pattern is at 5°C/min. The melting point onset should be within ±2.00°C of 36.5°C. If not, recheck calibration.
   *Note-*be certain to use identical capsules for the test portion as those used for reference standards and the instrument blank reference.
2. Melt each test portion completely and weigh 7 ± 0.200 mg of each test portion into the same kind of capsule used for the blank and reference samples (aluminium) and seal to minimize oxidation and other changes.
3. Place capsules in DSC at room temperature.
4. Heat rapidly to 80°C and hold for 10 min.
5. Cool to -60°C at 10°C/min and hold for 30 min.
6. Heat to 80°C at 5°C/min.
7. Use the baseline obtained for an empty capsule analysis from the final melt segment of the program to define the position of the baseline under the sample peaks. Overlay the final melting curve of the test portion over the curve for the empty capsule with a flexible ruler or other curve guide to define the baseline of the test portion back to where it intersects the initial deviation of the melting curve from its baseline. The baseline beneath the test portion should be a continuation of the baseline where there are no sample components present. If a shift has occurred in the heat capacity of the test portion after the melt, it will be evident relative to the baseline of the empty capsule. Have the instrument calculate the sigmoid baseline if it can, or connect the end of the peak point with the last point in which the test portion was in conjunction with the baseline of the empty capsule.

### Results

Determine the onset temperature in °C, which, if not computer generated, is an extrapolation to baseline of the steepest slope of the principal peak.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A solid compressed lubricating member for a razor cartridge comprising:
i) from 10% to 90% by weight of a particulate material comprising a water soluble polymer and
ii) from 1% to 40% by weight of a silicone polymer or mixtures thereof;
wherein at least a portion of said water soluble polymer is spray coated with said silicone polymer compound.

2. A solid compressed lubricating member for a razor cartridge according to claim 1, wherein said lubricating member comprises from 10% to 40%, preferably from 12% to 40%, more preferably from 12% to 30% by weight of said silicone polymer or mixture thereof.

3. A solid compressed lubricating member for a razor cartridge according to claim 1, wherein said silicone polymer or mixtures thereof are selected from dimethicones, cyclomethicones, phenyl trimethicones, trimethyl pentaphenyl trisiloxane, phenylated silicones and mixtures thereof.

4. A solid compressed lubricating member for a razor cartridge according to claim 1, further comprising a hydrophobic compound or mixtures thereof, wherein said hydrophobic compound or mixtures thereof is selected from natural oil or wax, synthetic oil or wax, trigylcerides, skin active agents, senates and mixtures thereof.

5. A solid compressed lubricating member for a razor cartridge according to claim 4, wherein said hydrophobic compound or mixtures thereof comprises capric and or caprylic triglycerides, grape seed oil, olive oil, shea butter, cocoa butter, lanolin, essential oils, peppermint oil, isohexadecane, petrolatum and mixtures thereof

6. A solid compressed lubricating member for a razor cartridge according to any one of the preceding claims, wherein said silicone polymer has a surface tension of 35 mN/m or less, preferably 30 mN/m or less, more preferably 25mN/m or less.

7. A solid compressed lubricating member for a razor cartridge according to any one of the preceding claims, wherein said silicone polymer has a melting point of less than 45°C, preferably less than 40°C, more preferably less than 30°C, most preferably 25°C or less.

8. A solid compressed lubricating member for a razor cartridge according to any one of the preceding claims, wherein said lubricating member has a bulk density of 1000kg/m³ to 1200kg/m³.

9. A solid compressed lubricating member for a razor cartridge according to any one of the preceding claims, wherein said particulate, has an average particle size of from 50 microns to 1250 microns.

10. A solid compressed lubricating member for a razor cartridge according to any one of the preceding claims, wherein said water soluble polymer comprises polyethylene oxide polymer.

11. A solid compressed lubricating member for a razor cartridge according to any one of the preceding claims, wherein at least 95%, preferably substantially 100% of said water soluble polymer is spray coated with said silicone polymer.

12. A solid compressed lubricating member according to claim 1, for a razor cartridge in the form of a tablet.

13. A hair removal cartridge comprising a lubricating member according to any one of the preceding claims.

14. A method of manufacturing a lubricating member according to claim 1, comprising the steps of
i) Providing a particulate of said water soluble polymer,
ii) Spray coating said silicone polymer onto said water soluble polymer particulates and
iii) Applying pressure onto said spray coated particulates to form a tablet.

15. The method according to claim 14, further comprising the steps of forming a liquid of said silicone polymer prior to said spray coating step.

16. The method according to claim 14, further comprising the steps of filling a pre-form with said particulates prior to the application of pressure and removing said compressed form from said pre-form.

## Patentansprüche

1. Festes gepresstes Schmierelement für eine Rasierkassette, umfassend:
**i)** 10-Gew.% bis 90Gew.-% eines Partikelmaterials, das ein wasserlösliches Polymer umfasst, und
**ii)** 1 Gew.-% bis 40 Gew.-% eines Silikonpolymers oder Gemische davon;
wobei mindestens ein Teil des wasserlöslichen Polymers mit dem Silikonpolymer sprühbeschichtet wird.

2. Festes gepresstes Schmierelement für eine Rasierkassette nach Anspruch 1, wobei das Schmierelement 10 Gew.-% bis 40 Gew.-%, bevorzugt 12 Gew.-% bis 40 Gew.-%, besonders bevorzugt 12 Gew.-% bis 30 Gew.-% des Silikonpolymers oder Gemisches davon umfasst.

3. Festes gepresstes Schmierelement für eine Rasierkassette nach Anspruch 1, wobei das Silikonpolymer oder die Gemische davon aus Dimethiconen, Cyclomethiconen, Phenyltrimethiconen, Trimethylpentaphenyltrisiloxan, phenylierten Siliconen und Gemischen davon ausgewählt sind.

4. Festes gepresstes Schmierelement für eine Rasierkassette nach Anspruch 1, das ferner eine hydrophobe Verbindung oder Gemische davon umfasst, wobei die hydrophobe Verbindung oder Gemische davon aus natürlichem Öl oder Wachs, synthetischem Öl oder Wachs, Triglyceriden, hautaktiven Mitteln, Wahrnehmungsstoffen und Gemische daraus ausgewählt ist.

5. Festes gepresstes Schmierelement für eine Rasierkassette nach Anspruch 4, wobei die hydrophobe Verbindung oder Gemische davon Caprin- und Capryltriglycerid, Traubenkernöl, Olivenöl, Sheabutter, Kakaobutter, Lanolin, ätherische Öle, Pfefferminzöl, Isohexandecan, Petrolatum und Gemische davon umfasst.

6. Festes gepresstes Schmierelement für eine Rasierkassette nach einem der vorhergehenden Ansprüche, wobei das Silikonpolymer eine Oberflächenspannung von 35 mN/m oder darunter, bevorzugt 30 mN/m oder darunter, besonders bevorzugt 25mN/m oder darunter aufweist.

7. Festes gepresstes Schmierelement für eine Rasierkassette nach einem der vorhergehenden Ansprüche, wobei das Silikonpolymer einen Schmelzpunt von weniger als 45 °C, bevorzugt weniger als 40 °C, besonders bevorzugt weniger als 30 °C, ganz besonders bevorzugt 25 °C oder weniger aufweist.

8. Festes gepresstes Schmierelement für eine Rasierkassette nach einem der vorhergehenden Ansprüche, wobei das Schmierelement eine Schüttdichte von 1000 kg/m³ bis 1200kg/m³ aufweist.

9. Festes gepresstes Schmierelement für eine Rasierkassette nach einem der vorhergehenden Ansprüche, wobei das Partikelmaterial eine durchschnittliche Partikelgröße von 50 Mikrometern bis 1250 Mikrometern aufweist.

10. Festes gepresstes Schmierelement für eine Rasierkassette nach einem der vorhergehenden Ansprüche, wobei das wasserlösliche Polymer Polyethylenoxid-Polymer umfasst.

11. Festes gepresstes Schmierelement für eine Rasierkassette nach einem der vorhergehenden Ansprüche, wobei mindestens 95 %, bevorzugt im Wesentlichen 100 % des wasserlöslichen Polymers mit dem Silikonpolymer sprühbeschichtet ist.

12. Festes gepresstes Schmierelement nach Anspruch 1 für eine Rasierkassette in Form einer Tablette.

13. Haarentfernungskassette, die ein Schmierelement nach einem der vorhergehenden Ansprüche umfasst.

14. Verfahren zum Herstellen eines Schmierelements nach Anspruch 1, umfassend die Schritte des
i) Vorsehens eines Partikelmaterials aus dem wasserlöslichen Polymer,
**ii)** Sprühbeschichten des Silikonpolymers auf die wasserlöslichen Polymerpartikel und
**iii)** Anwenden von Druck auf die sprühbeschichteten Partikel um eine Tablette zu formen.

15. Verfahren nach Anspruch 14, das ferner die Schritte des Bildens einer Flüssigkeit aus dem Silikonpolymer vor dem Sprühbeschichtungsschritt umfasst.

16. Verfahren nach Anspruch 14, das ferner die Schritte des Füllens einer Vorform mit den Partikeln vor dem Anwenden von Druck und Entfernens der gepressten Form von der Vorform umfasst.

## Revendications

1. Elément de lubrification comprimé solide pour une cartouche de rasoir comprenant :
i) de 10 % à 90 % en poids d'une matière particulaire comprenant un polymère soluble dans l'eau ; et
ii) de 1 % à 40 % en poids d'un polymère de silicone ou de mélanges de celui-ci ;
au moins une partie dudit polymère soluble dans l'eau étant revêtu par pulvérisation par ledit composé de polymère de silicone.

2. Elément de lubrification comprimé solide pour une cartouche de rasoir, selon la revendication 1, dans lequel ledit élément de lubrification comprend de 10 % à 40 %, de préférence de 12 % à 40 %, de façon davantage préférée de 12 % à 30 % en poids, dudit polymère de silicone ou du mélange de celui-ci.

3. Elément de lubrification comprimé solide pour une cartouche de rasoir, selon la revendication 1, dans lequel ledit polymère de silicone ou les mélanges de celui-ci sont choisis parmi les diméthicones, les cyclométhicones, les phényl triméthicones, le triméthyl pentaphényl trisiloxane, les silicones phénylés et les mélanges de ceux-ci.

4. Elément de lubrification comprimé solide pour une cartouche de rasoir, selon la revendication 1, comprenant en outre un composé hydrophobe ou les mélanges de celui-ci, dans lequel ledit composé hydrophobe ou les mélanges de celui-ci sont choisis parmi une huile ou cire naturelle, une huile ou cire synthétique, les triglycérides, les agents agissant sur la peau, les agents sensoriels et les mélanges de ceux-ci.

5. Elément lubrifiant comprimé solide pour une cartouche de rasoirs, selon la revendication 4, dans lequel ledit composé hydrophobe ou les mélanges de celui-ci comprennent les triglycérides capriques et/ou capryliques, l'huile de pépins de raisin, l'huile d'olive, le beurre de karité, le beurre de cacao, la lanoline, les huiles essentielles, l'huile de menthe poivrée, l'isohexadécane, la vaseline et leurs mélanges.

6. Elément de lubrification comprimé solide pour une cartouche de rasoir, selon l'une quelconque des revendications précédentes, dans lequel ledit polymère de silicone a une tension superficielle de 35 mM/m ou moins, de préférence de 30 mM/m ou moins, de façon davantage préférée de 25 mM/m ou moins.

7. Elément de lubrification comprimé solide pour une cartouche de rasoir, selon l'une quelconque des revendications précédentes, dans lequel ledit polymère de silicone a un point de fusion de moins de 45°C, de préférence de moins de 40°C, de façon davantage préférée de moins de 30°C, de la façon que l'on préfère le plus de 25°C ou moins.

8. Elément de lubrification comprimé solide pour une cartouche de rasoir, selon l'une quelconque des revendications précédentes, dans lequel ledit élément de lubrification a une masse volumique apparente de 1 000 kg/m³ à 1 200 kg/m³.

9. Elément de lubrification comprimé solide pour une cartouche de rasoir, selon l'une quelconque des revendications précédentes, dans lequel ladite matière particulaire a une dimension moyenne de particule de 50 microns à 1 250 microns.

10. Elément de lubrification comprimé solide pour une cartouche de rasoir, selon l'une quelconque des revendications précédentes, dans lequel ledit polymère soluble dans l'eau comprend un polymère de poly(oxyde d'éthylène).

11. Elément de lubrification comprimé solide pour une cartouche de rasoir, selon l'une quelconque des revendications précédentes, dans lequel au moins 95 %, de préférence sensiblement 100 % dudit polymère soluble dans l'eau, sont revêtues par pulvérisation par ledit polymère de silicone.

12. Elément de lubrification comprimé solide selon la revendication 1, pour une cartouche de rasoir, sous la forme d'un comprimé.

13. Cartouche de rasage comprenant un élément de lubrification selon l'une quelconque des revendications précédentes.

14. Procédé de fabrication d'un élément de lubrification selon la revendication 1, comprenant les étapes consistant à :
i) se procurer des particules dudit polymère soluble dans l'eau ;
ii) appliquer en revêtement par pulvérisation ledit polymère de silicone sur lesdites particules de polymère soluble dans l'eau ; et
iii) appliquer une pression sur lesdites particules revêtues par pulvérisation pour former un comprimé.

15. Procédé selon la revendication 14, comprenant en outre les étapes de formation d'un liquide dudit polymère de silicone avant ladite étape d'application en revêtement par pulvérisation.

16. Procédé selon la revendication 14, comprenant en outre les étapes de remplissage d'une préforme par lesdites particules avant l'application de pression et le retrait de ladite forme comprimée à partir de ladite préforme.
